# EUROPÄISCHE PATENTANMELDUNG

(11) **EP 0 521 435 A2**
(43) Veröffentlichungstag der Anmeldung: **07.01.1993**
(21) Anmeldenummer: 92110991.4
(22) Anmeldetag: 29.06.1992
(51) Int. Cl.: C12N 5/04, C12P 1/00

(54) **Verfahren zur Elicitierung von Sekundärstoffen in pflanzlichen Zellkulturen**

(30) Priorität: 04.07.1991 DE 4122208
(71) Anmelder: B.A.T. Cigarettenfabriken GmbH, D-20354 Hamburg (DE)
(72) Erfinder: Zenk, Meinhard, Prof. Dr., W-8000 München 60 (DE); Kutchan-Zenk, Toni, Dr., W-8000 München 60 (DE); Gundlach, Heidrun, W-8046 Garching (DE)
(74) Vertreter: Schwabe - Sandmair - Marx

(57) **Zusammenfassung**

Die Erfindung betrifft ein Verfahren zur Elicitierung von Sekundärstoffen in pflanzlichen Zellkulturen, bei dem man der Zellkultur eine Verbindung der folgenden allgemeinen Formel (I) zu einer Endkonzentration von 0,1 µmol/l bis 1 mmol/I zusetzt. 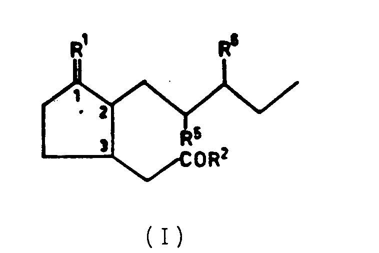
Durch den Zusatz einer Verbindung der allgemeinen Formel (I) wird nicht nur die Produktion von Sekundärstoffen neu induziert, sondern auch die Ausbeute wesentlich gesteigert.

## Beschreibung

Die Erfindung betrifft ein Verfahren zur Elicitierung, d. h. Induktion der Bildung von Sekundärstoffen in pflanzlichen Zellkulturen.

Es ist bekannt, daß Pflanzen aktiv auf eine Infektion oder andere Einflüsse aus der Umgebung durch Synthese von bestimmten Abwehrstoffen reagieren. Dieses Phänomen kann in Pflanzenzellkulturen durch Verbindungen hervorgerufen werden, die als Elicitoren bekannt sind. Bei diesen Elicitoren handelt es sich in vielen Fällen um Kohlenhydrate von Pilzen. Darüber hinaus sind jedoch Glykoproteine, einfache und komplexe Kohlenhydrate, Fettsäuren und andere nicht biologische Verbindungen als Elicitoren bekannt (vgl. Allen G. Darvill et al., Ann. Rev. Plant Physiol. 1984, 35, 243; Cell Culture and Somatic Cell Genetics of Plants, Eds. Constable Friedrich, Vasil K. Indra, Academic Press, San Diego, Californien, 1987, Seite 153 bis 196).

Die Möglichkeit, durch gezielte Induktion in Pflanzenzellkulturen die Produktion von wirtschaftlich relevanten Stoffen zu induzieren, erfuhr in letzter Zeit ein zunehmendes Interesse. Zu den Sekundärstoffen, die durch gezielte Induktion in Pflanzenzellkulturen produziert werden können, sind beispielsweise zu nennen: Atropin und Scopolamin, Colchicin, Emetin, Codein und Morphium, Chinidin und Chinin, Reserpin und andere. Weitere Beispiele sind die Cardenoloide, Lignane, Gossipol, Hyoscyamin, Reserpin sowie weitere in der Parfüm- und Kosmetikindustrie verwendbare Substanzen.

Die Erzeugung von pflanzlichen Sekundärstoffen über die Fermentation von pflanzlichen Zellkulturen wird durch den Umstand behindert, daß viele Zellkulturen nicht in der Lage sind, im entdifferenzierten Zustand Sekundärstoffe spontan, auch bei Zusatz von Elicitoren, zu produzieren. Bislang wurden ausschließlich Elicitoren mikrobiellen Ursprungs, wie Zellwände oder Glucane verwendet. Der Nachteil dieser Präparate liegt darin, daß manche pflanzliche Zellkulturen nur auf ganz spezifische mikrobielle Zellwandkomponenten ansprechen, so daß eine Verallgemeinerung des Elicitierungsprozesses durch Einsatz von ein und denselben mikrobiellen Zellwandkomponenten nicht möglich ist. Ein weiterer Nachteil der bisher verwendeten Elicitoren liegt darin, daß diese drastischen Aktivitätsschwankungen unterliegen und somit nur begrenzt verwendet werden können.

Jasmonsäure und Derivate davon sind als Inhaltsstoffe des Jasminöls bekannt. Es ist auch bekannt, daß bestimmte Stereoisomere der Jasmonsäure bei einigen Pflanzen wachstumsregulatorische Funktionen besitzen. Aus der DD-PS 221 059 ist ein jasmonsäurehaltiges Mittel zur Reifebeschleunigung, Ertragsverbesserung und Ertragsstabilisierung bei Zuckerrohr bekannt.

Aus der DD-PS 209 379 sind Mittel, die Auxine und/oder Jasmonsäure enthalten, zur Regulation der Blüteperiode bei Kaffee bekannt.

Die Anmelderin hat nun überraschend gefunden, daß durch Zusatz eines Jasmonsäurederivates entsprechend der nachfolgenden allgemeinen Formel (I) zu pflanzlichen Zellkulturen verschiedensten Ursprungs die Sekundärstoffproduktion induziert werden kann und eine drastisch erhöhte Steigerung der Sekundärstoffproduktion erzielt werden kann.

Erfindungsgemäß wird daher ein Verfahren zur Elicitierung von Sekundärstoffen in pflanzlichen Zellkulturen zur Verfügung gestellt, bei dem man eine Verbindung der folgenden Formel (I) 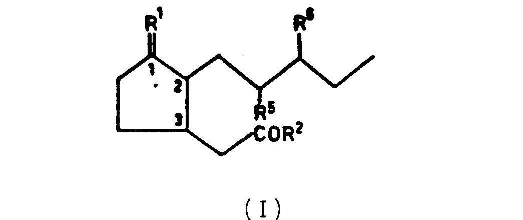
in der R¹ O, H oder OH bedeutet, R² OH, OM (M ist Erdalkali- oder Alkalimetall oder NH₄), NHR³ oder OR⁴ bedeutet, wobei R³ H, C, -4-Acyl, Cₗ -₄ -Alkyl, Phenyl- oder Naphthyl bedeutet und R⁴ C ₁-₄-Alkyl, Phenyl oder Naphthyl ist, R⁵ und R⁶ jeweils für H oder gemeinsam für eine Doppelbindung stehen und die Substituenten am C-1-, C-2-und C-3-Atom a- oder β-ständig angeordnet sind, oder ein Salz, einen Ester oder ein Aminosäure-oder Kohlenhydratderivat davon der Zellkultur zusetzt.

Das erfindungsgemäße Verfahren umfaßt alle stereoisomeren Verbindungen der Formel (I) und Mischungen davon.

Unter einer C₁ -₄-Alkylgruppe ist eine gerad-oder verzweigtkettige Alkylgruppe mit 1 bis 4 C-Atomen wie Methyl, Ethyl, Propyl und Butyl zu verstehen.

Unter einem Erdalkali- oder Alkalimetall sind beispielsweise Na, K und Ca zu verstehen.

Unter einer C₁ -₄-Acylgruppe sind gerad- oder verzweigtkettige Carbonsäurereste mit 1 bis 4 C-Atomen zu verstehen.

In den bevorzugten Verbindungen der Formel (I) bedeuten R¹ O, R² COOCH₃ oder OH und R⁵ und R⁶ bilden eine Doppelbindung oder bedeuten jeweils H.

Besonders bevorzugte Verbindungen der Formel (I) sind jene, in denen R¹ O, R² COOCH₃ und R⁵ und R⁶ H bedeuten; R¹ O, R² OH ist und R⁵ und R⁶ eine Doppelbindung bilden; R¹ OH, R² OH ist und R⁵ und R⁶ eine Doppelbindung bilden; R¹ O, R² OH ist und R⁵ und R⁶ H bedeuten.

Der Verbindungen der Formel (I) können auch mit einem geeigneten Kohlenhydratrest, z. B. Glucopyranosyl, oder mit Aminosäuren derivatisiert werden. Die Herstellung derartiger Derivate wird beispielsweise von O. Miersch et al., Z. Chemie 27, 261 (1987) und von R. Kramell et al., Tetrahedron, Vol. 44, No. 18, 5791 (1988) beschrieben.

Die Verbindung der Formel (I) wird in einem geeigneten organischen Lösungsmittel, z. B. Ethanol, gelöst und zu einer Endkonzentration zwischen 0,1 umol/1 und 1 mmol/I der Zellkultur zugesetzt.

Ein bevorzugter Konzentrationsbereich liegt zwischen 10 umol/1 und 500 umol/l. Ein besonders bevorzugter Konzentrationsbereich liegt zwischen 25 umol/1 und 500 umol/l.

Der Zusatz einer Verbindung der Formel (I) zu den pflanzlichen Zellkulturen beeinflußt das Wachstum der Kulturen nicht, führt jedoch zu einer wesentlichen Steigerung der Produktion von Sekundärstoffen.

Durch das erfindungsgemäße Verfahren können daher niedrig produzierende Zellkulturlinien in hochproduzierende, industriell wertvolle Stämme umgewandelt werden.

Das erfindungsgemäße Verfahren ist generell einsetzbar, unabhängig von der Pflanzenzellkultur. Das Anlegen der Pflanzenzellkultur erfolgt nach bekannten Verfahren und Techniken und ist in den einschlägigen Lehrbüchern beschrieben (z. B. Cell Culture and Somatic Cell Genetics of Plants, Ed.: Indra K. Vasil, Academic Press, Inc., New York, London, Tokyo (1984)). Die Zellkulturen können beispielsweise von Pflanzen der Familie Lauraceae, Ranunculaceae, Papaveraceae, Caryophyllaceae, Polygonaceae, Brassicaceae, Tiliaceae, Malvaceae, Rosaceae, Caesalpiniaceae, Fabaceae, Rutaceae, Apiaceae, Rhamnaceae, Primulaceae, Ericaceae, Gentianaceae, Apocynaceae, Rubiaceae, Solanaceae, Scrophulariaceae, Lamiaceae, Valerianaceae, Asteraceae, Convallariaceae, Hyacinthaceae, Alliaceae, Asphodelaceae, Colchicaceae, Zingiberaceae, Poaceae und Coniferophytina hergestellt werden.

Bevorzugt sind Pflanzenzellkulturen der Familien Fabaceae, Rutaceae, Apocynaceae, Rubiaceae, Solanaceae und Zingiberaceae.

Besonders bevorzugt sind Pflanzenzellkulturen der Familien Apocynaceae und Solanaceae.

Beispiele für Sekundärstoffe, die durch das erfindungsgemäße Verfahren produziert werden können, sind: Atropin und Scopolamin, isoliert aus Hyoscyamus- und Datura-Wurzelkulturen, Colchicin aus Herbstzeitlose, Emetin aus Zell- und Wurzelkulturen der Brechwurz, Codein und Morphin aus Zellkulturen des Schlafmohns, Chinidin und Chinin aus Zellkulturen von Cinchona succirubra bzw. Cinchona ledgeriana, Ajmalin, Rescinamin, Reserpin aus Zellkulturen von Rauwolfia-Arten. Diese Substanzen werden arzneilich eingesetzt. Insbesondere sind also die Stoffgruppen der Indolalkaloide, der Isochinolinalkaloide, der Lignane und Isoprenoide zu nennen.

Weitere Beispiele sind: Digitalis purpurea: Cardenoloide, die unter dem Einfluß von Jasmonat gegenüber der Kontrolle eine 2-fach gesteigerte Konzentration erreichen; Linum hirsutum: Lignane, die 2,5-fach gesteigert sind; Gossypium herbaceum: Gossipol, 10-fach gesteigert gegenüber der Kontrolle; isolierte Wurzelkulturen von Hyoscyamus alba: Hyoscyamin/Scopolamin: stark parasympatholytische bzw. zentraldämpfende, medizinisch wertvolle Alkaloide, 12-fache Steigerung gegenüber der Kontrolle; Zellkulturen von Colchicum autumnale, die das Gichtmittel Colchicin produzieren, 8-fache Steigerung gegenüber der Kontrolle; Zellkulturen von Cephaelis ipecacuanha, die das arzneilich genutzte Emetin produzieren, das bei Keuchhusten und Bronchialasthma sowie als erbrechungsförderndes Mittel Anwendung findet, 7,5-fache Steigerung gegenüber der Kontrolle; Zellkulturen von Cinchona succirubra bzw. Cinchona ledgeriana, die medizinisch als Antiarhythmikum bzw. als Chemotherapeutikum (Malaria) eingesetzt werden, Steigerung 14-fach; Zellkulturen von Rauwolfia serpentina, die die therapeutisch genutzten Indolalkaloide Reserpin (blutdrucksenkend, sedierend), Serpentin (blutdrucksenkend), Ajmalin (bei Herzrhythmusstörungen), Ajmalicin (Gehirn-durchblutungsfördernd), Yohimbin (durchblutungsfördernd) produzieren.

Die folgenden Beispiele zeigen anhand ausgewählter Zellkulturen die wesentliche Steigerung der Sekundärstoffproduktion durch das erfindungsgemäße Verfahren.

Pflanzliche Zellkulturen, deren differenzierte Ausgangspflanzen zur Synthese eines gewünschten Pflanzenstoffes befähigt sind, werden in flüssigem Nährmedium gezogen. Diese Medien können entweder literaturbekannte Medien sein (vgl. Cell Culture and Somatic Cell Genetics of Plants, Band 1, Laboratory Procedures and their Applications, Herausgeber: Indra K. Vasil, Academic Press, New York (1984)) oder auch modifizierte Medien, die in Laboratorien optimiert wurden, um beste Wachstums- oder Produktionsbedingungen zu erhalten. Die Zellkultur wird in Flüssigmedium kultiviert und kann entweder in entsprechenden Kulturgefäßen oder in Fermentern herangezogen werden. Nach einer Anwachsphase von 2 bis 10 Tagen wird dem Medium eine in Ethanol gelöste Verbindung der Formel (I) zugesetzt. Um die Löslichkeit der Verbindung der Formel (I) zu erhöhen, können Derivate wie z. B. Glukoseester, Aminosäure- oder andere Kohlenhydrataddukte eingesetzt werden. Nach Bildung der maximalen Konzentration an Sekundärstoffen wird der Versuch durch Separierung der Zellen vom Medium abgebrochen und die Sekundärstoffe werden anschließend nach bekannten Verfahren aus dem Zellüberstand oder den Zellen isoliert.

### Beispiel 1

Einer Zellsuspensionskultur von Eschscholtzia californica, die in 1 Liter LS-Nährmedium (Linsmaier, E. M., Skoog, F., Physiol. Plant 18, 100 (1965)) herangewachsen ist und deren Frischgewicht 200 g beträgt, wird Methyljasmonat zu einer Endkonzentration von 20 umol/1 zugesetzt. Die Kultur wird für 5 Tage weiterkultiviert, anschließend mit 200 ml salzsaurem Ethanol (1,7 ml HCI konz./1 I Ethanol) 2 Stunden lang bei 60 ° C extrahiert. Der so gewonnene methanolische Extrakt wird einer HPLC-Chromatographie unterzogen und zeigt gegenüber einer nicht mit Methyljasmonat behandelten Kontrolle eine 15-fache Steigerung der Benzophenanthridin-Produktion.Die Gesamtausbeute der Kultur beträgt 300 mg Benzophenanthridine pro Liter Kulturmedium. Die entsprechende, nicht mit Methyljasmonat behandelte Kultur ergibt 18 mg pro Liter Gesamtbenzophenanthridine.

### Beispiel 2

Eine Kultur von Glycine max wird mit 330 g Frischgewicht in 1 Liter 4x-Medium (Gamborg, Miller, Ojima, Experiment Research 50, 151 - 158 (1968)), 2,4-D 2 mg/I, NES 0,5 mg/I, IES 0,5 mg/I, Kinetin 0,2 mg/I versetzt. 3 Tage später wird Methyljasmonat zu einer Endkonzentration von 250 umol/1 zugesetzt. Nach einer Kultivierungsperiode von 6 Tagen wird die Kultur mit Methanol extrahiert (50 g Frischgewicht/200 ml Methanol; 2 Stunden, 60 ° C). Der Extrakt enthält im Vergleich zu nicht elicitierten Kontrollen eine jeweils ca. 30-fach höhere Konzentration an Daidcein- und Genistein-Glycosiden.

### Beispiel 3

Eine Kultur von Rauwolfia serpentina, in LS-Medium 3 Tage gewachsen, wird zu einer Endkonzentration von 200 umol/1 mit Jasmonsäure versetzt und 4 Tage weiterkultiviert. Der methanolische Extrakt des Gewebes enthält eine 8-fache Vallesiacotamin-Konzentration gegenüber der Kontrolle. Der Absolutwert ist 70 mg/I Medium.

### Beispiel 4

Eine Kultur von Rubia tinctorum wird in LS-Medium kultiviert. Nach 4 Tagen werden der Kultur 2',3'-Dihydrojasmonat zugesetzt. Nach 7 Tagen wird die Kultur geerntet und der Anthrachinon-Gehalt bestimmt. Der Zuwachs an Alizarin beträgt das ca. 40-fache gegenüber der Kontrolle.

### Beispiel 5

Eine 2 Tage alte Kultur von Ruta chalepensis (4x-Medium) wird mit Jasmonatglukosid zu einer Endkonzentration von 40 µmol/l versetzt, für 5 Tage weiterkultiviert und mit Methanol extrahiert. Der Extrakt zeigt einen 18-fachen Rutacridon-Gehalt gegenüber der Kontrolle.

### Beispiel 6

Einer 4 Tage alten Rauwolfia canescens Zellkultur (LS-Medium) wird 3-Hydroxyjasmonat zu einer Endkonzentration von 200 µmol/l zugesetzt. Die Kultur wird 6 Tage weiterkultiviert und mit einer entsprechenden Kontrolle, der kein 3-Hydroxyjasmonat zugesetzt wurde, verglichen. Dabei zeigt sich, daß in dieser Zellkultur 6 Indolalkaloide gebildet werden, die in der Kontrollkultur nicht vorkommen.

Dies zeigt, daß Sekundärstoffe, die in der Ausgangskultur nicht gebildet werden, durch das erfindungsgemäße Verfahren neu induziert werden können.

Dieses Phänomen wurde bei Suspensionskulturen von Lycopersicum esculentum, Nicotiana tabacum, Nicotiana plumbagenifolium, Ruta graveolens, Magnolia denudata und Weigelia florida ebenfalls beobachtet.

### Beispiel 7

Eine Zellkultur von Lactuca sativa wird 24 Stunden nach dem Überimpfen in LS-Medium mit 3-Hydroxyjasmonsäure zu einer Endkonzentration von 250 µmol/l versetzt. Die Extraktion mit Methanol erfolgt 6 Tage später. Die elicitierten Kulturen enthalten das Custonolid Lettucenin A in einer Konzentration von ca. 30 mg/I. In den Extrakten einer Kontrollkultur ist Lettucenin A nicht nachweisbar.

### Beispiel 8

Zellkulturen von Eschscholtzia californica werden - wie in Beispiel 1 beschrieben - mit Methyljasmonat elicitiert. Nach Zugabe des Jasmonats werden im Abstand von je 1 Stunde Aliquots entnommen und die m-RNA extrahiert. Die m-RNA wird mit einer Probe gegen das Berberinbrückenenzym, ein Schlüsselenzym des Benzophenanthridin-Stoffwechsels, hybridisiert. Der Vergleich mit einer entsprechenden Kontrollkultur zeigt, daß sich in der Zellkultur, die mit Jasmonat versetzt wurde, die spezifische m-RNA-Menge in 2 Stunden verdoppelt, in 10 Stunden weiter ansteigt und dann wieder abnimmt. Die so induzierte m-RNA ist ein Indiz für die Genaktivierung der an der Elicitierung und an der Alkaloidbiosynthese beteiligten Enzyme. Die Verbindungen der Formel (I) sind demnach in der Lage, reprimierte Sekundärstoffgene zu aktivieren.

## Patentansprüche

1. Verfahren zur Elicitierung von Sekundärstoffen in pflanzlichen Zellkulturen durch Zusatz eines Elicitors, dadurch gekennzeichnet, daß der Elicitor eine Verbindung der allgemeinen Formel (I) ist: 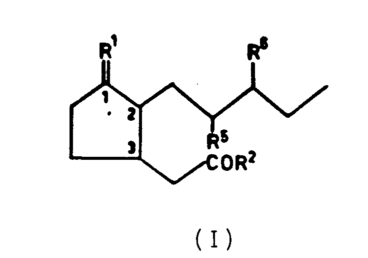
in der R¹ O, H oder OH bedeutet, R² OH, OM (M ist Erdalkali- oder Alkalimetall Oder NH₄), NHR³ oder OR⁴ bedeutet, wobei R³ H, C₁₋₄₋ Acyl, C₁₋₄-Alkyl, Phenyl- oder Naphthyl bedeutet und R⁴ C1-5-Alkyl, Phenyl oder Naphthyl ist, R⁵ und R⁶ jeweils für H oder gemeinsam für eine Doppelbindung stehen und die Substituenten am C-1-, C-2- und C-3-Atom a- oder β-ständig angeordnet sind, oder ein Salz, einen Ester oder ein Aminosäure-oder Kohlenhydratderivat davon.

2. Verfahren nach Anspruch 1, bei dem in der Formel (I) R¹ O, R² COOCH₃ oder OH und R⁵ und R⁶ eine Doppelbindung oder H bedeuten.

3. Verfahren nach Anspruch 1 oder 2, dadurch gekennzeichnet, daß der Elicitor eine Verbindung der allgemeinen Formel (I) ist, in der R¹ O, R² COOCH₃ und R⁵ und R⁶ H bedeuten oder R¹ O, R² OH ist und R⁵ und R⁶ eine Doppelbindung bilden oder R¹ OH, R² OH ist und R⁵ und R⁶ eine Doppelbindung bilden oder R¹ O, R² OH ist und R⁵ und R⁶ H bedeuten.

4. Verfahren nach einem der Ansprüche 1 bis 3, dadurch gekennzeichnet, daß der Elicitor der Zellkultur zu einer Endkonzentration von 0,1 µmol/I bis 1 mmol/I zugesetzt wird.

5. Verfahren nach Anspruch 4, dadurch gekennzeichnet, daß der Elicitor der Zellkultur zu einer Endkonzentration von 10 µmol/l bis 500 µmol/ I zugesetzt wird.

6. Verfahren nach Anspruch 5, dadurch gekennzeichnet, daß der Elicitor der Zellkultur zu einer Endkonzentration von 25 µmol/l bis 500 µmol/l zugesetzt wird.

7. Verfahren nach einem der vorhergehenden Ansprüche 1 bis 6, dadurch gekennzeichnet, daß die Zellkultur aus Pflanzen der Familie der Fabaceae, Rutaceae, Apocynaceae, Rubiaceae, Solanaceae und Zingiberaceae hergestellt wird.

8. Verfahren nach Anspruch 7, dadurch gekennzeichnet, daß die Zellkultur aus Pflanzen der Familie der Apocynaceae und Solanaceae hergestellt wird.

9. Sekundärstoff aus pflanzlichen Zellkulturen, dadurch gekennzeichnet, daß dieser durch Elicitierung mit einer Verbindung der Formel (I) gemäß Anspruch 1 bis 6 in einer Zellkultur aus Pflanzen gemäß Anspruch 7 oder 8 produziert worden ist.
